# EUROPEAN PATENT SPECIFICATION

(11) **EP 4 035 651 B1**
(45) Date of publication and mention of the grant of the patent: **27.11.2024**
(21) Application number: 21719184.0
(22) Date of filing: 11.01.2021
(51) Int. Cl.: A61K 8/365, A61K 8/60, A61K 8/67, A61Q 19/02

(54) **DEPIGMENTING DERMOPHARMACEUTICAL OR COSMETIC COMPOSITION AND USE THEREOF**
DEPIGMENTIERENDE DERMOPHARMAZEUTISCHE ODER KOSMETISCHE ZUSAMMENSETZUNG UND DEREN VERWENDUNG
COMPOSITION DERMOPHARMACEUTIQUE OU COSMÉTIQUE DÉPIGMENTANTE ET SON UTILISATION

(43) Date of publication of application: 03.08.2022
(73) Proprietor: Mesoestetic Pharma Group, S.L., 08840 Viladecans (Barcelona) (ES)
(72) Inventor: MARTÍNEZ GUTIÉRREZ, Alfredo, 08840 Viladecans (BARCELONA) (ES); GONZÁLEZ RODRÍGUEZ, María del Carmen, 08840 Viladecans (BARCELONA) (ES); LUIS GARCÍA, Luis Shotze, 08840 Viladecans (BARCELONA) (ES)
(74) Representative: Hernández Hernández, Carlos
(86) International application number: PCT/ES2021/070005
(87) International publication number: WO 2022/064083

(56) References cited:
- US-A1- 2006 045 896
- US-A1- 2020 215 145

## Description

The present invention is disclosed in the appended claims and relates to a depigmenting dermopharmaceutical or cosmetic composition and to its use for removing or at least whitening melanic spots on skin.

More specifically, in a first aspect, the invention relates to the non-therapeutic use of a depigmenting dermopharmaceutical or cosmetic composition comprising a combination of retinol, diosmin and ferulic acid, which act synergistically to inhibit melanogenesis, in addition to adequate excipients for the topical formulation thereof.

In a second aspect, the invention also relates to the combination of retinol diosmin and ferulic acid for use in a dermopharmacological composition for use in the prevention and treatment of diseases and conditions related to the overproduction or uneven distribution of melanin, wherein said use comprises topically administering a therapeutically effective amount of said combination to a person who needs it.

The colour of human skin is determined by different factors such as race, sex, phototype, seasonality and epidermal melanin concentration, although its complete physiological mechanism has not been fully determined.

The melanocytes present in the basal layer of the epidermis produce melanins inside intramelanocytary organelles called melanosomes. Melanins are complex structure polyphenolic homogeneous biopolymers whose colour ranges from brown to black (eumelanin) and from red to yellow (pheomelanin). These melanins (pheomelanins and eumelanins) are transported to nearby keratinocytes to be distributed in their interior and act as a protective screen against ultraviolet radiation.

Melanogenesis is a defense mechanism of the skin and melanin biosynthesis is stimulated by ultraviolet light. Thus, the physiological function of tanning as a consequence of the impact of ultraviolet light on the skin is to protect it from this ultraviolet radiation.

Skin pigmentation disorders consisting of hyperproduction of melanin or hypermelanosis and melanin deficiency or hypomelanosis are frequent. Hypermelanosis appears more frequently in certain periods of life, usually caused by exogenous factors such as overexposure to sun. Hypermelanosis is characterised by the appearance of dark and coloured spots on the skin, particularly in exposed areas, which give them a certain degree of visual heterogeneity; it is generally an aesthetic problem. These spots are caused by melanocytic hypertrophy or by a greater accumulation of melanin in the keratinocytes located in the superficial part of the epidermis.

Similarly, different dysfunctions of melanogenesis due to the effect of exogenous aggressions, for example hormonal or ageing alterations, trigger the appearance of dark, brown or blackish spots of melanic origin in the form of melasmas, ephelides, senescence spots, lentigo, etc.

The ability to modify the natural pigmentation of the skin is a widespread desire among the European, Asian and American community, in aspects such as clarification, whitening, depigmentation, removal of age spots, etc. For this reason, controlling pigmentation using exogenous agents is a cause for high social and commercial demand.

The melanin formation mechanism or melanogenesis is highly complex and occurs mainly in melanosomes. The biochemical precursor of melanin is the amino acid L-tyrosine, which can be formed by hydroxylation of phenylalanine, an essential amino acid found in the human body.

On the one hand, tyrosinase (EC 1.14 18.1), which is found in the interior of melanosomes, acts as a hydroxylase, transforming L-Tyrosine into L-Dihydroxyphenylalanine or L-DOPA. On the other, tyrosinase enzymatically catalyses the transformation of L-DOPA into dopaquinone, since this enzyme can also act as an oxidase, depending on the substrate. In order to be active, an enzymatic maturing process is required under the action of certain cellular factors.

Dopaquinone is transformed into dopachrome, which can follow two metabolic pathways: one that leads to the formation of eumelanins, black in colour, and another that leads to the formation of yellow pheomelanins, of a yellowish colour. Both coexist in the skin and their proportion determines its colour or phototype.

Three key enzymes participate in this melanogenesis process: tyrosinase, a limiting enzymatic glycoprotein which is found in melanosome membranes; Tyrosinase-related protein 1 or TRP 1 and Tyrosinase-related protein 2 or TRP 2, glycoproteins which are also located in melasome membranes and complement tyrosinase activity.

Once the melanins are formed in the melanosomes, a mechanical transfer of the melanins formed to the epidermal keratinocytes must take place. There is a complex tubular system and cellular factors that contribute to take the granules of the melanins to the cellular extensions of the melanocytes.

These cellular cytoplasmatic extensions form a network that comes into contact with the keratinocytes, forming the so-called melanocitary unit, such that a melanocyte comes into contact with around 36 keratinocytes. The intracellular melanins already situated in melanocytic extensions are transferred to the keratinocytes. The melanin granules are apically disposed in the keratinocytes such as to confer greater protection against the deleterious effects of UV light

A powerful stimulus for stimulating melanogenesis is irradiating the skin with ultraviolet light, which immediately causes an increase in melanocitary cAMP. This increase activates the cAMP/PKA/CREB pathway, which in turn activates the microphthalmia-associated transcription factor or MITF, the main regulator of the genes involved in melanin synthesis (*TYR, TRP1, TRP2,* etc.), being tyrosinase the main enzyme involved in melanogenesis.Thus, since tyrosinase plays a key role in the melanin production process, inhibitors of this enzyme are often used as skin depigmenting agents.

Tyrosinase plays an essential role in melanin synthesis, due to the fact that it directly regulates the melanin produced, while other enzymes only modify the type of melanin that is synthesised in the biochemical pigmentation route. Therefore, its inhibition has become one of the most important strategies in the development of new depigmenting agents and has gained great importance in medical and cosmetic products. Pharmacological tyrosinase inhibitors or other melanogenic pathways may serve *de novo* as topical melanogenesis inhibitors, with effect on skin whitening. The use of skin whitening products varies between the different cultures and races across the globe. Therefore, the use of genuine therapies ranges from their use in skin diseases to beauty products.

Therefore, many studies related to tyrosinase and its inhibitors have been carried out in the last century and continue to date (Gerardo M. Casañola-Martín, Yovani Marrero-Ponce, Huong Le-Thi-Thu, Mahmud Tareq Hassan Khan, Francisco Torrens, Antonio Rescigno and Concepción Abad, The tyrosinase enzyme: 2. Inhibitors of natural and synthetic origin, AFINIDAD LXX, 564, October - December 2013).

Both synthetic and natural inhibitors of melanin formation in the melanogenesis process are disclosed in literature. A major group of darkening inhibitors consists of structurally analogous compounds with phenolic substrates. These generally show competitive inhibition by these substrates, although such inhibition may vary depending on the type of enzyme and the substrate used (Mayer, A. M.; Harel, E. In Phenoloxidases and Their Significance in Fruit and Vegetables; Fox, P. F., Ed.; Elsevier: London, 1991, pp. 373-398).

Products containing hydroquinone in a percentage of 2% to 4% are moderately effective, but may be cytotoxic to melanocytes and potentially mutagenic for mammal cells. Since 2000, the use of hydroquinone as a depigmenter in cosmetic products is prohibited in the European Union, where its use is limited to pharmaceutical specialties sold under medical prescription for products containing high percentages of hydroquinone (4%) and without medical prescription for products with a lower percentage of the active ingredient (2%). Resorcinol is an isomer of hydroquinone which was also once used as a depigmenter, but its use in cosmetics is also currently prohibited.Other hydroquinone-derived active ingredients, such as arbutin, and fungi-isolated inhibitors, such as kojic acid, have very relative effectiveness (Curto, E. V.; Kwong, C.; Hermersdorfer, H.; Glatt, H.; Santis, C.; Virador, V.; Hearing, V. J., Jr.; Dooley, T. P. Biochem Pharmacol 1999, 57, pp. 663-72).

Many melanin inhibitors are resorcinol derivatives or polyphenol derivatives from flavonoids or trans-stilbene, such as resveratrol or its derivatives. These types of compounds are known to form strong chelates with metal ions. Various compounds based on the fraction of resorcinol have been used as inhibitors of melanin formation. Few compounds have been reported as being potent inhibitors of melanin in the skin.

In EP 1857109 A2, for example, phenylthiourea derivatives are disclosed as mammal tyrosinase inhibitors in cellular-free melanocytes or mammal melanoma cell extracts. EP 2117498 B1 discloses 4-hydroxyphenoyacetic acid derivatives for clearing and/or whitening the skin and/or for reducing pigmentation and/or for reducing hyperpigmentation and/or for inhibiting melanogenesis.

Furthermore, the melanogenic gene expression is controlled by a complex network which involves transcription factors and other intracellular signalling pathways. Genetic, biochemical and pharmacological evidence has established that the signalling of pro-opiomelanocortin (POMC) peptides, the stimulating Alpha-melanocyte hormone (α-MSH) and the adrenocorticotropic hormone (ACTH) via the c-AMP pathway plays a decisive role in the differentiation of melanocytes and in the regulation of melanogenesis.

This pathway begins with POMC produced by the pituitary gland and epidermal keratinocytes in α-MSH. α-MSH functions in the same way as all hormones, adhering to a specific coupled transmembrane protein receptor called α-MSH-1 Receptor (MC1R) in nearby melanocytes.

By binding to MC1R, α-MSH stimulates the α subunit of the stimulating G protein. This protein activates adenylate cyclase, increasing the level of intracellular c-AMP. The increase in the level of c-AMP acts as a second messenger for activating A kinase protein (AKP), which penetrates the melanocyte nucleus and activates the cAMP Response Element-Binding Protein (CREB) transcription factor, which finally induces the expression of the Microphthalmia Transcription factor (MITF).

MITF is a "master regulator" of the melanocyte function and melanogenesis. It regulates the expression of a large number of genes involved in the survival, differentiation, mobility and production of melanocyte melanin, including tyrosinase (TYR) and other related melanogenic enzymes, and tyrosinase-related proteins 1 and 2 (TYRP1 and TYRP2).

In order to become functional proteins, these melanogenic enzymes will undergo a maturation process through the Golgi apparatus, protein processing, covalent modifications, chaperone binding, oligomerisation and traffic. These melanogenic enzymes travel through the secretory tract and early endosome intermediates. From there they are sent to the preformed stage II melanosomes, where they initiate the synthesis of pigments and subsequent maturation.

Furthermore, Takaaki Yamada et al. (in "Wnt/β-Catenin and Kit Signalling Sequentially Regulate Melanocyte Stem Cell Differentiation in UVB-Induced Epidermal Pigmentation", Journal of Investigative Dermatology (2013) 133, 2753-2762) demostrated that Wnt/β-catenin signalling regulates the differentiation of melanocyte stem cells (McSC - Melanocyte Stem Cells) in the epidermal melanocytes induced by UVB radiation.

Also, oxidative stress, mainly induced by ultraviolet radiation, plays an important role in skin ageing and in the appearance of different melanogenesis dysfunctions. Thus, there is evidence that chronological skin ageing and UV radiation share molecular pathways, including the alteration in the transduction of signals that promote the expression of matrix metalloproteinases (MMP), with the ensuing reduction in procollagen and connective tissue damage (Callaghan TM, et al., "A review of ageing and examination of clinical methods in the assessment of ageing skin. Part I: Cellular and molecular perspectives of skin ageing", Int J Cosmet Sci 2008; 30:313-322). UV-B radiation causes skin erythema, called sunburn, induced by ROS (reactive oxygen species) which stimulate the synthesis of prostaglandin E2 through COX-2 excretion, finally promoting the inflammation process (Masaki H., "Role of antioxidants in the skin: Anti-ageing effects", J Dermatol Sci 2010; 58:85-90). In addition to different inflammatory mediators, including prostanoids, cytokins and chemiocins, reactive oxygen species are released in the epidermal layer during the inflammatory process, stimulating the melanocytes to increase melanin production. In skin melanosis, the inflammation alters the basal layer, causing the melanin to be released and trapped in the macrophages of the papillary dermis (pigmentary incontinence), which gives the skin a grey-purple-brown colour.

US 2020/215145 A1 (MITCHELL SHANE [AU] ET AL) discloses a method for preventing, improving or treating a skin condition in a subject, wherein the condition *isinter alia* dark spots, age spots, mottled pigmentation, skin pigmentation, melasma, darkened skin, dark circles under the eyes and changes associated with skin ageing. Examples of the compositions to be used in this method comprises extracts from sugar cane. The polyphenols contained in these extracts can be inter alia diosmin and ferulic acid (see par. [0151]). Additionally the compositions may contain retinol or retinol derivatives (see par. [0220])

Based on the different approaches and causes of the previously described melanogenesis disorders, following is a summary of the causes involved in skin hyperpigmentation and whose research has resulted in the subject matter of the present invention:
- Wnt/β-catenin signalling pathway;
- Oxidative stress caused by reactive oxygen species (ROS);
- Activation of alpha-melanocyte stimulating hormone (α-MSH);
- Pigmentation induced by inflammation; and
- Regulation of the Microphthalmia Transcription Factor (MITF) for the production of melanocyte melanin.

The depigmenting dermopharmaceutical or cosmetic composition used in the present invention comprises a combination of retinol, diosmin and ferulic acid, which act synergistically to inhibit melanogenesis, in addition to adequate excipients for the topical administration thereof.

Following is an explanation of the individual involvement in melanogenesis of each of the compounds contained in the combination of the invention.

Retinol, a retinoic acid precursor, can induce inhibitor proteins of the Wnt/β-catenin pathway, for example DAB2 (disabled homolog 2) (see Li, J., et al., Wnt/beta-Catenin Signalling Pathway in Skin Carcinogenesis and Therapy. Biomed Res Int, 2015. 2015: p. 964842). Retinoic acid activates the non-canonical Wnt signalling pathway while inhibiting the canonical pathway. The Wnt signalling pathway plays a role in pluripotency and differentiation, depending on the state of activation of the canonical (which requires the nuclear accumulation of β-catenin and its interaction with transcription factor 7 (TCF7)/binding factor of the lymphoid enhancer factor-1 (LEF1), in Wnt-responsive elements and participates in the maintenance of embryonic endothelial stem cells) and non-canonical pathways (through the actions of the E2-α transcription factor, TCF3 can antagonise the canonical pathway) (see Osei-Sarfo, K. y L.J. Gudas, "Retinoic acid suppresses the canonical Wnt signalling pathway in embryonic stem cells and activates the non-canonical Wnt signalling pathway", Stem Cells, 2014. 32(8): pp. 2061-71).

Retinoic acid or retinol inhibits melanin synthesis cells stimulated by the α melanocyte-stimulating hormone (α-MSH or 3-isobutyl-1-methylxantin (IBMX)) (see Sato, K., et al., "Depigmenting mechanisms of all-trans retinoic acid and retinol on B16 melanoma cells", Biosci Biotechnol Biochem, 2008. 72(10): pp. 2589-97).

Although it is possible for the retinoic acid and the retinol to affect other melanogenic pathways, the main effect of the retinoic acid and retinol is via cAMP-dependent melanogenic pathways. α-MSH and IBMX increase the concentration cAMP, which activates the microphthalmia-associated transcription factor (MITF) and then MITF binds to and activates the melanogenic gene promoters, resulting in greater melanin synthesis (supra).

It has also been demonstrated that retinoic acid has an antiapoptotic and antioxidant effect on different cells (see Barbara Ahlemeyer et al. "Retinoic acid reduces apoptosis and oxidative stress by preservation of SOD protein level", Free Radical Biology and Medicine, 2001. Volume 30 (Issue 10): pp. 1067-1077).

Furthermore, ferulic acid, a powerful scavenger of free radicals (ROS, reactive oxygen species), attenuates oxidative stress, which is responsible for reducing high arterial pressure by enhancing the endothelial function and facilitating greater bioavailability of the nitric oxide in the arterial vasculature (see Alam, M.A., "Anti-hypertensive Effect of Cereal Antioxidant Ferulic Acid and Its Mechanism of Action", Front Nutr, 2019. 6: p. 121). Similarly, it has low toxicity and many physiological functions (anti-inflammatory, antioxidant, antimicrobial, anticancer and antidiabetic), and has a protective effect on the main skin structures: keratinocytes, fibroblasts, collagen, elastin. It also inhibits melanogenesis, enhances angiogenesis and accelerates wound healing. It is widely applied in skin care formulations as a photoprotective agent, as a retardant in skin photoageing processes and as an illuminating component. However, its use is limited by its tendency to easily oxidise (see, for example, Zdunska, K., et al., "Antioxidant Properties of Ferulic Acid and Its Possible Application", Skin Pharmacol Physiol, 2018. 31(6): pp. 332-336). In the experimental results, Jeong-Hwa Choi et al., "Anti-melanogenic effects of Hordeum vulgare L. barley sprout extract in murine B16F10 melanoma cells" (Journal of Nutrition and Health, 2019. vol: 52 (2): p. 168), showed that treatment with barley sprout water extract (with polyphenols, including p-Coumaric, ferulic and vanillic acid) reduced the production of cellular melanin. The molecular findings supported the fact that the suppressed activity and expression of tyrosinase and MITF proteins by the barley sprout water extract is associated with a reduction in cellular melanogenesis. Additionally, the antimelanogenic effect of the barley sprout water extract is similar to that of arbutin, a whitening agent frequently used in cosmetic.

As regards diosmin (also known as venosmin), it is a natural flavonoid whose properties as a vascular protection agent are known, being administered orally to treat chronic venous insufficiency. It also has free radical-eliminating, antioxidant and anti-inflammatory properties (Boisnic, S., et al., "Anti-inflammatory and antiradical effects of a 2% diosmin cream in a human skin organ culture as model", J Cosmet Dermatol, 2018. 17(5): pp. 848-854). Its properties as an antihyperglycemic, antimutagenic and antiulcerous agent have also been demonstrated (Feldo, M., et al., "Influence of Diosmin Treatment on the Level of Oxidative Stress Markers in Patients with Chronic Venous Insufficiency", Oxid Med Cell Longev, 2018. 2018: p. 2561705; Hasan, H.F., et al., "Diosmin attenuates radiation-induced hepatic fibrosis by boosting PPAR-gamma expression and hampering miR-17-5p-activated canonical Wnt-beta-catenin signalling", Biochem Cell Biol, 2017. 95(3): pp. 400-414).

Given the aforementioned known properties of the individual components of the combination, the present inventors found that, surprisingly, the topical administration of the composition of the invention, which comprises a combination of diosmin, retinol and ferulic acid, has an enhanced synergistic depigmenting action compared to the depigmenting action of each of these components administered separately and even sequentially.

In the context of the present invention, "depigmenting action" is understood to be a skin depigmenting activity due to the interaction of the composition of the invention with one or more of the aforementioned mechanisms of melanogenesis.

In a preferred embodiment, in the dermopharmaceutical or cosmetic composition of the invention, which comprises a combination of retinol, diosmin and ferulic acid, the combination of said components is present in a range of concentration of 0.3 to 30% by weight with respect to the weight of the composition. In one preferred embodiment, the combination is present in a range of 10 to 30% by weight.

In another embodiment, the retinol, diosmin and ferulic acid are present in the combination in a range of concentration of 0.1 to 8%, of 0.1 to 10% and of 0.1 to 10%. In one preferred embodiment, the retinol, diosmin and ferulic acid are present in the combination in a range of concentration of 3.0 to 8%, of 2.0 to 10% and of 5.0 to 10%, in all cases by weight of the composition.

As mentioned earlier, the dermopharmaceutical or cosmetic composition used in the present invention includes adequate excipients for its formulation for topical administration. Such excipients can be selected from oily solvents, anhydrous solvents, hydroalcoholic solvents, aqueous solvents, loads, preservatives, gelling agents, perfumes, surfactants, sunscreens, antioxidants, viscosizers, emulsifiers, silicones and any of the ingredients used in the field of cosmetics or dermatology in the usual concentrations, provided that said excipients do not interfere with the depigmenting activity of the combination hereby disclosed. The composition of the invention can be formulated in any galenic or cosmetic form of those normally used for topical administration, for example in the form of an aqueous, hydroalcoholic, hydroglycolic or oil solution. It can also be included in oil-in-water or water-in-oil emulsions or in a multiple emulsion or formulated as a serum, a foam or an aerosol.

In a second aspect, the invention relates to the combination of retinol, diosmin and ferulic acid for use in preventing and treating diseases and conditions related to the overproduction or uneven distribution of melanin, said use comprising the topical administration of an effective amount of said combination to a person who needs it.

In this context, diseases and affections related to the overproduction or uneven distribution of melanin includes, but not limited to, hyperpigmentation spots caused by skin ageing, melasma, liver diseases, thermal burns and topical wounds, skin pigmentation due to inflammatory conditions of fungal, microbial and viral origin, vitiligo, carcinoma, melanoma, post-inflammatory hyperpigmentation and similar.

Below, the invention is illustrated by means of the following illustrative and non-limiting examples thereof and in reference to the attached figures, wherein:
Fig. 1A: Shows the depigmenting effect in the pathways involved in skin hyperpigmentation of each compound separately and of the combination of the invention.
Fig. 1B: Shows the depigmenting effect of each compound separately and of the combination of the invention.
Fig. 2: Shows a graphic representation of the most probable collaborative mechanism of the combination of the invention.
Fig. 3: Shows a graph representing the results of in vitro assay, percentage of the tyrosinase activity in untreated cells (control), cells stimulated with 2 mM L-tyrosine (stimulated), cells stimulated with 2 mM L-tyrosine and treated with 10 µM retinol during the first 24 hours, 5 µM tyrosine during the following 24 hours and 1 mM ferulic acid during the last 24 hours (additive) and cells stimulated with 2 mM L-tyrosine and treated with 10 µM retinol, 5 µM diosmin and 1 mM ferulic acid for 3 days.

### Examples

### Synergistic effect of the combination of the invention on skin depigmentation

Firstly, a study was carried out to evaluate 12 skin depigmenting compounds with the aim of predicting which of the combinations of the different compounds has a synergistic effect, in order to subsequently identify a collaborative action mechanism among those selected.

To this end, a protein-level molecular characterisation of pathophysiological mechanisms involved in skin hyperpigmentation was carried out, determining the five main molecular pathways that regulate skin melanin synthesis:
a) Wnt/β-catenin signalling
b) Oxidative stress by ROS - SCF
c) Activation of α-MSH
d) Pigmentation induced by inflammation
e) MITF regulation for melanin production.

In parallel, the compounds were evaluated at protein level according to all their molecular targets described in different scientific databases (Supertarget, Stitch, Drugbank, Pubmed) and official sources (AEMS, EMA, FDA).

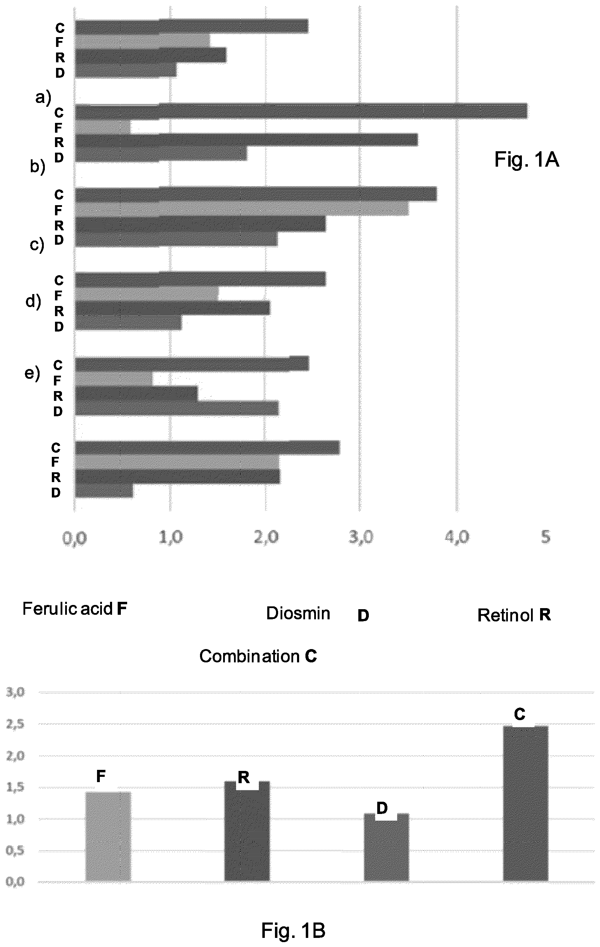

With these data, a mathematical model was created crossing all the targets in order to evaluate which of the compounds could have a synergistic effect. To this end, an artificial neuronal network (ANN) was used which provided a value or score that quantified the probability of each combination of molecular targets having a synergistic effect, considering only the values with the highest scores (>78). Five combinations of compounds with synergistic effects were obtained in three of the five molecular pathways that regulate skin pigmentation (a, c and d) and a combination of compounds which have synergistic effects on four of the five molecular pathways (a, b, c and d). These results are summarised in Table 1 below and shown in figure 1A. Table 1 shows whether any of the target proteins of these compounds show a synergistic effect in each molecular skin pigmentation pathway, with at least one score higher than 78% (p < 0.1). The number of the different target combinations which have a synergistic effect is shown in parentheses.

**Table 1**

| Combination of compounds | Path a) | Path b) | Path c) | Path d) | Path e) |
|---|---|---|---|---|---|
| Ferulic acid + retinol | >78% (1) | <71% | >78% (1) | >78% (27) | <71% |
| Ferulic acid + diosmin | <71% | <71% | <71% | Yes (1) | <71% |
| Retinol + diosmin | >78% (1) | >78% (1) | >78% (1) | >78% (13) | <71% |

Secondly, the collaborative mechanism was characterized at molecular level wherethrough the combination of retinol, ferulic acid and diosmin exerts synergy in skin depigmentation.

To this end, a sampling method was used based on a mathematical algorithm (Sampling Methods) that describes and represents the points of the molecular pathways wherethrough the synergistic effect occurs between the compounds, thereby elucidating the exact action mechanism that explains said synergy. Briefly, this method identifies the possible action mechanisms that lead to a physiological response (in this case the inhibition of hyperpigmentation) when the system is stimulated with a specific stimulus (here the combination of the invention).

This is how the ability of the compounds (individually or combined) to reverse the protein alterations produced in skin hyperpigmentation mechanisms, i.e. the ability of each compound and of the combination to activate the proteins which are inhibited in skin hyperpigmentation and vice versa, was determined calculating the intensity of the depigmenting effect of each compound and combination using a mathematical model that measures the proportion of effector proteins known for their role in hyperpigmentation which are modulated by the compounds. The results are shown in figure 1B.

As can be observed in Fig. 1A and 1B, the combination of the compounds retinol, diosmin and ferulic acid enables a more pronounced induction in all the skin depigmentation mechanisms than any of the compounds separately. Additionally, each compound has different and complementary depigmenting properties, underlining the importance of using this specific combination of diosmin, retinol and ferulic acid.

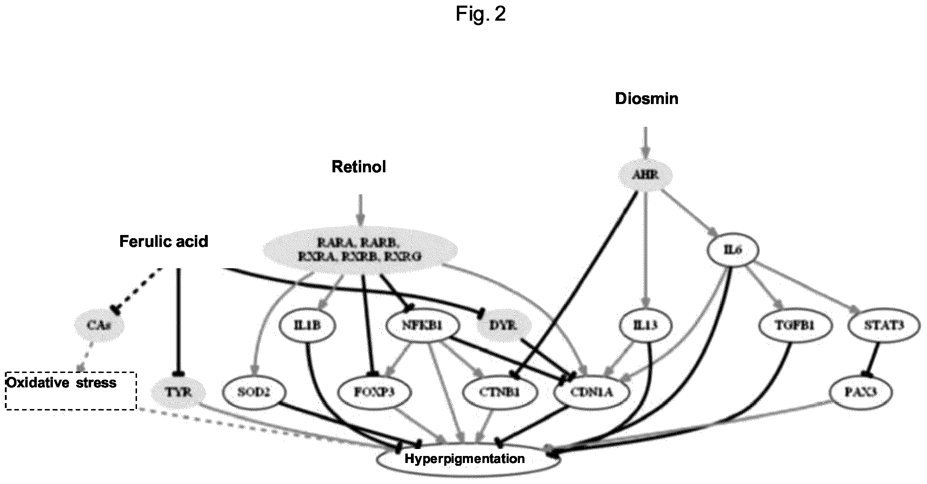

Based on mathematically modelled and biologically contextualized methods, the most probable collaborative depigmentation pathways of the combination described herein. Thus, mathematical analysis provides a description of the molecular pathways and key proteins involved in skin depigmentation. Thus, mathematical analysis provides a description of the molecular pathways and key proteins involved in the skin depigmentation caused by the combination. These mechanisms show that the activity of the individual components of the combination is interconnected, supporting the presence of a synergistic effect between them (fig. 2).

As can be observed in figure 2, ferulic acid, through its direct inhibition of TYR (tyrosinase, TYR, a limiting enzyme for controlling melanin production) and DYR (dihydropholate reductase), induces a subregulation of melanin synthesis and melanogenesis by induction of melanocyte senescence (CDN1A, increased activity of p21, involved in the suppression of melanogenesis by induction of melanocyte senescence). It also underlines an indirect effect of its inhibition of Cas (carbonic anhydrases) on pigmentation induced by oxidative stress (the inhibition of these enzymes reduces cellular oxidative stress and the production of ROS in mitochondria). Retinol, through the induction of RAR and RXR (retinoic acid receptors), promotes a reduction of NF-kB signaling (NFKB1, CDN1A, reducing the proliferation of keratinocytes and melanocytes induced by UV radiation), of WNT/β signaling (CTNB1), of oxidative stress (SOD2) and of hyperpigmentation induced by inflammation (IL1B, an anti-melanogenic cytokin known to inhibit skin pigmentation). Lastly, diosmin, via AHR (aryl hydrocarbide receptor, a transcription activator bound to a ligand which is bound to the promoting region of different inflammatory mediators), induces the expression of melanogenic cytokins (IL6, IL13, TGFB1) and inhibits wNT/β pathway signaling (CTNB1) and melanogenesis of the skin (p21, PAX3, reducing the gene expression of MITF).

### In vitro assay on human melanocytes which shows the synergistic effect of the combination

An in vitro assay was performed on human melanocytes treated with the combination and with the compounds separately to confirm the existence of a synergistic effect between them. Additionally, the cells were treated with L-tyrosine to stimulate melanogenic activity therein. L-tyrosine, in addition to being a substrate of the tyrosinase enzyme, key in melanin synthesis, is capable of activating cellular receptors which activate melanogenesis regulator signaling pathways (Slominski A, et al., "L-tyrosine and L-dihydroxyphenylalanine as hormone-like regulators of melanocyte functions", Pigment Cell Melanoma Res. 2012; 25(1): 14-27). This condition was aimed at stimulating melanogenesis in the melanocytes in order to test the effect of the active ingredients, given that melanocytes in normal culture medium have low basal activity and this would not make it possible to evaluate the possible inhibition of the active ingredients within a few days.

### Experimental design

For the experiment, human melanocytes were grown in culture medium (#PCS-200-013, ATCC) under different conditions for 3 days. The experiment includes four different conditions:
1) Melanocytes grown in culture medium (control).
2)Melanocytes grown in culture medium with 2 mM L-tyrosine. L-tyrosine was added to these melanocytes every 24 hours until the end of the experiment (stimulated).
3)Melanocytes grown in culture medium with 2mM L-tyrosine, 10 µM retinol, 5 µM diosmin and 1 mM ferulic acid every 24 hours successively. In this case, 2 mM L-tyrosine + 10 µM retinol were added during the first 24 hours; next, the culture medium was removed and the cells were washed with PBS; next, 2 mM L-tyrosine + 5 µM diosmin were added during the following 24 hours and the cells were washed with PBS; lastly, 2 mM L-tyrosine + 1mM ferulic acid were added during the last 24 hours until completing the 3 days (additive effect).
4)Melanocytes grown in culture medium with 2 mM L-tyrosine with 10 µM retinol, µM diosmin and 1 mM ferulic acid in combination. In this case, 2 mM L-tyrosine + 10 µM retinol + 5 µM diosmin + 1 mM ferulic acid were added every 24 hours until completing the 3 days (combination effect).

These conditions are summarized in Table 2 below:

| | Day 1 | Day 2 | Day 3 |
|---|---|---|---|
| 1) Control | - | - | - |
| 2) Stimulated | 2 mM L-Tyr | 2 mM L-Tyr | 2 m M L-Tyr |
| 3) Additive effect | 2 mM L-Tyr + 10 µM retinol | 2 mM L-Tyr + 5 µM diosmin | 2 mM L-Tyr + 1 mM ferulic acid |
| 4) Combination | 2 mM L-Tyr + 10 µM retinol + 5 µM diosmin + 1 mM ferulic acid | 2 mM L-Tyr + 10 µM retinol + 5 µM diosmin + 1 mM ferulic acid | 2 mM L-Tyr + 10 µM retinol + 5 µM diosmin + 1 mM ferulic acid |

At the end of the treatment, the cells were trypsinised and lysed in PBS pH 7.0 with 1% Triton X-100. The cell lysate, which contains tyrosinase and other enzymes of the molecular melanogenesis pathway, was incubated together with L-DOPA and MBTH (Winder AJ y Harris H, "New assays for the tyrosine hydroxylase and dopa oxidase activities of tyrosinase", 1991; 198(2): 317-26). L-DOPA acts like a substrate of tyrosinase in the enzyme reaction and MBTH is a compound that binds to the product of the tyrosinase reaction, generating a colour complex. This mixture was incubated at room temperature for 1 hour and the absorbance quantified at 492 nm using a spectrophotometer.

In parallel, part of the cell lysate was used to quantify the total amount of protein through the BCA assay. This assay uses a reagent that binds to the proteins, generating a colour product, whose absorbance can be quantified using spectrophotometry. This quantification makes it possible to normalize the results of the enzymatic assay to eliminate the differences in the total amount of proteins that there can be between the samples, thereby verifying that the differences observed are not due to differences in the amount of proteins in the sample.

### Results

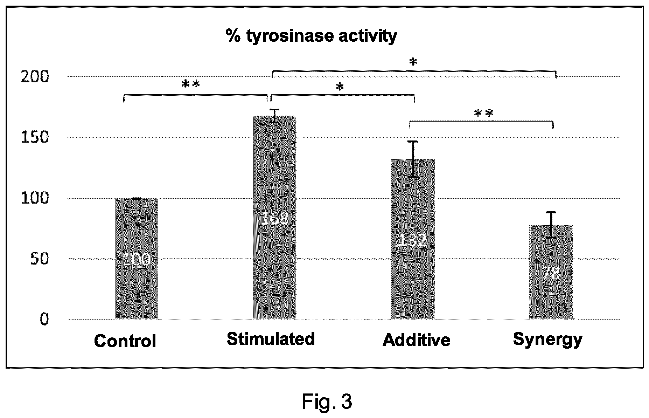

The results obtained are shown in figure 3. As can be observed, stimulation with L-tyrosine increases tyrosinase activity to 168% with respect to the non-stimulated control. With regard to treatment with the active ingredients, adding each of the active ingredients successively and separately decreases tyrosinase activity to 132%, while adding the three active ingredients together decreases tyrosinase activity to 78%. All these changes are statistically significant. Therefore, it can be observed that the combination of the active ingredients is more effective than adding each of them successively to inhibit tyrosinase activity in human melanocyte cultures.

### Example of a composition of the invention in the form of a cream

| Components | % by weight |
|---|---|
| Ferulic acid | 5 |
| Diosmin | 2 |
| Retinol | 3 |
| Glycerine | 4 |
| Butilene glycol | 2 |
| Disodium EDTA | 0.1 |
| Phenoxyethanol ethylhexyl glycerine | 1 |
| Xanthane gum | 0.2 |
| Acrylate/ C10-30 alkyl acrylate cross-linked polymers | 0.3 |
| Sodium hydroxide | 0.4 |
| Cetylic alcohol, glyceryl stearate, PEG-75 stearate, Ceteth-20, Steareth-20 | 4 |
| Cetearyl alcohol | 2 |
| Jojoba esters, Helianthus annus Weed Wax, Acacia decurrens Flower | 1 |
| Wax, Polyglycerin-3 | |
| PEG-8, tocopherol, ascorbyl palmitate, ascorbic acid, citric acid | 0.5 |
| Caprylic/capric acid triglycerides | 3 |
| Dimethicone | 2 |
| Coconut caprate/caprylate, unsaponifiable hydrogenated olive oil | 2 |
| Isodecyl neopentanoate | 2 |
| Tocopheryl acetate | 0.5 |
| Rice starch | 2 |
| Perfume | 0.3 |
| Water | Up to 100% |

## Claims

1. The non-therapeutic use of a depigmenting dermopharmaceutical or cosmetic composition comprising a combination of retinol, diosmin and ferulic acid to inhibit melanogenesis, which acts synergistically, in addition to adequate excipients for the topical formulation thereof.

2. The non-therapeutic use of a depigmenting dermopharmaceutical or cosmetic composition according to claim 1, **characterised in that** the combination of retinol, diosmin and ferulic acid is present in a range of concentration of 0.3 to 30% by weight of the composition.

3. The non-therapeutic use of a depigmenting dermopharmeceutical or cosmetic composition according to claim 2, **characterised in that** the combination of retinol, diosmin and ferulic acid is present in a range of concentration of 10 to 30% by weight of the composition.

4. The non-therapeutic use of a depigmenting dermopharmeceutical or cosmetic composition according to any of the preceding claims, **characterised in that** the retinol, diosmin and ferulic acid are present in the combination in a range of concentration of 0.1 to 8%, of 0.1 to 10% and of 0.1 to 10%, respectively, by weight of the composition.

5. The non-therapeutic use of a depigmenting dermopharmeceutical or cosmetic composition according to claim 4, **characterised in that** the retinol, diosmin and ferulic acid are present in the combination in a range of concentration of 3.0 to 8%, of 2.0 to 10% and of 5.0 to 10%, respectively, by weight of the composition.

6. A combination of retinol, diosmin and ferulic acid for use in a dermopharmacological composition for treating diseases and conditions related to the overproduction or uneven distribution of melanin, said use comprising topical administration to a person who needs an effective amount of said combination.

7. The combination of retinol, diosmin and ferulic acid for use according to claim 6, **characterised in that** the retinol, diosmin and ferulic acid are present in the combination in a range of concentration of 0.1 to 8%, of 0.1 to 10% and of 0.1 to 10%, respectively, by weight of the composition.

8. The combination of retinol, diosmin and ferulic acid for use according to claim 7, **characterised in that** the retinol, diosmin and ferulic acid are present in the combination in a range of concentration of 3.0 to 8%, of 2.0 to 10% and of 5.0 to 10%, respectively, by weight of the composition.

## Patentansprüche

1. Nicht-therapeutische Verwendung einer
depigmentierenden dermopharmazeutischen oder kosmetischen Zusammensetzung, umfassend eine Kombination aus Retinol, Diosmin und Ferulasäure zur Hemmung der Melanogenese, die synergistisch wirkt, sowie geeignete Hilfsstoffe für deren topische Formulierung.

2. Nicht-therapeutische Verwendung einer
depigmentierenden dermopharmazeutischen oder kosmetischen Zusammensetzung nach Anspruch 1,
**dadurch gekennzeichnet, dass** die Kombination aus Retinol, Diosmin und Ferulasäure in einem Konzentrationsbereich von 0,3 bis 30 Gew.-% der Zusammensetzung vorhanden ist.

3. Nicht-therapeutische Verwendung einer
depigmentierenden dermopharmazeutischen oder kosmetischen Zusammensetzung nach Anspruch 2,
**dadurch gekennzeichnet, dass** die Kombination aus Retinol, Diosmin und Ferulasäure in einem Konzentrationsbereich von 10 bis 30 Gew.-% der Zusammensetzung vorhanden ist.

4. Nicht-therapeutische Verwendung einer
depigmentierenden dermopharmazeutischen oder kosmetischen Zusammensetzung nach einem der vorhergehenden Ansprüche, **dadurch gekennzeichnet, dass** das Retinol, das Diosmin und die Ferulasäure in der Kombination in einem Konzentrationsbereich von 0,1 bis 8 %, von 0,1 bis 10 % bzw. von 0,1 bis 10 % vorhanden sind, bezogen auf das Gewicht der Zusammensetzung.

5. Nicht-therapeutische Verwendung einer
depigmentierenden dermopharmazeutischen oder kosmetischen Zusammensetzung nach Anspruch 4; **dadurch gekennzeichnet, dass** das Retinol, das Diosmin und die Ferulasäure in der Kombination in einem Konzentrationsbereich von 3,0 bis 8 %, von 2,0 bis 10 % bzw. von 5,0 bis 10 % vorhanden sind, bezogen auf das Gewicht der Zusammensetzung.

6. Kombination aus Retinol, Diosmin und Ferulasäure zur Verwendung in einer dermopharmakologischen Zusammensetzung zur Behandlung von Krankheiten und Beschwerden, die mit der Überproduktion oder ungleichmäßigen Verteilung von Melanin zusammenhängen, wobei die Verwendung die topische Verabreichung an eine Person umfasst, die eine wirksame Menge der Kombination benötigt.

7. Kombination aus Retinol, Diosmin und Ferulasäure zur Verwendung nach Anspruch 6, **dadurch gekennzeichnet, dass** das Retinol, Diosmin und die Ferulasäure in der Kombination in einem Konzentrationsbereich von 0,1 bis 8 %, von 0,1 bis 10 % bzw. von 0,1 bis 10 % vorhanden sind, bezogen auf das Gewicht der Zusammensetzung.

8. Kombination aus Retinol, Diosmin und Ferulasäure zur Verwendung nach Anspruch 7, **dadurch gekennzeichnet, dass** das Retinol, das Diosmin und die Ferulasäure in der Kombination in einem Konzentrationsbereich von 3,0 bis 8 %, von 2,0 bis 10 % bzw. von 5,0 bis 10 % vorhanden sind, bezogen auf das Gewicht der Zusammensetzung.

## Revendications

1. L'usage non thérapeutique d'une
composition dermopharmaceutique ou cosmétique dépigmentante comprenant une combinaison de rétinol, de diosmine et d'acide férulique pour inhiber la mélanogénèse, qui agit en synergie, ainsi que des excipients adéquats pour sa formulation topique.

2. L'usage non thérapeutique d'une
composition dermopharmaceutique ou cosmétique dépigmentante selon la revendication 1,
**caractérisée par le fait que** la combinaison de rétinol, de diosmine et d'acide férulique est présente dans une fourchette de concentration de 0,3 à 30 % en poids de la composition.

3. L'usage non thérapeutique d'une
composition dermopharmaceutique ou cosmétique dépigmentante selon la revendication 2,
**caractérisée par le fait que** la combinaison de rétinol, de diosmine et d'acide férulique est présente dans une fourchette de concentration de 10 à 30 % en poids de la composition.

4. L'usage non thérapeutique d'une
composition dermopharmaceutique ou cosmétique dépigmentante selon l'une des quelconques revendications précédentes, **caractérisée par le fait que** le rétinol, la diosmine et l'acide férulique sont présents en combinaison dans une fourchette de concentration de 0,1 à 8 %, de 0,1 à 10 % et de 0,1 à 10 %, respectivement, en poids de la composition.

5. L'usage non thérapeutique d'une
composition dermopharmaceutique ou cosmétique dépigmentante selon la revendication 4,
**caractérisée par le fait que** le rétinol, la diosmine et l'acide férulique sont présents en combinaison dans une fourchette de concentration de 3,0 à 8 %, de 2,0 à 10 % et de 5,0 à 10 %, respectivement, en poids de la composition.

6. Une combinaison de rétinol, de diosmine et d'acide férulique pour un usage dans une composition dermopharmacologique visant à traiter les maladies et affections liées à la surproduction ou à la distribution inégale de mélanine, ledit usage comprenant l'administration topique à une personne nécessitant une quantité efficace de ladite combinaison.

7. Une combinaison de rétinol, de diosmine et d'acide férulique pour un usage selon la revendication 6,
**caractérisée par le fait que** le rétinol, la diosmine et l'acide férulique sont présents en combinaison dans une fourchette de concentration de 0,1 à 8 %, de 0,1 à 10 % et de 0,1 à 10 %, respectivement, en poids de la composition.

8. Une combinaison de rétinol, de diosmine et d'acide férulique pour un usage selon la revendication 7,
**caractérisée par le fait que** le rétinol, la diosmine et l'acide férulique sont présents en combinaison dans une fourchette de concentration de 3,0 à 8 %, de 2,0 à 10 % et de 5,0 à 10 %, respectivement, en poids de la composition.
